# EUROPEAN PATENT SPECIFICATION

(11) **EP 4 178 640 B1**
(45) Date of publication and mention of the grant of the patent: **15.05.2024**
(21) Application number: 21733566.0
(22) Date of filing: 14.06.2021
(51) Int. Cl.: A61M 1/00, A61F 13/02

(54) **FOAM MANIFOLD PAD**
LEITUNGSSYSTEMSCHAUMSTOFFKISSEN
COUSSINET DISTRIBUTEUR EN MOUSSE

(30) Priority: 09.07.2020 US 202063049866 P
(43) Date of publication of application: 17.05.2023
(73) Proprietor: KCI Manufacturing Unlimited Company, Dublin, D01C4E0 (IE)
(72) Inventor: SIMMONS, Tyler H., San Antonio, Texas 78265 (US); HANER, Marilyn, San Antonio, Texas 78265 (US); GONZALEZ, Javier, San Antonio, Texas 78265 (US)
(74) Representative: Simmons & Simmons
(86) International application number: PCT/IB2021/055226
(87) International publication number: WO 2022/008998

(56) References cited:
- WO-A1-2017/063036
- WO-A1-2020/014178
- US-A1- 2018 289 558
- US-A1- 2019 307 935

## Description

### CROSS-REFERENCE TO RELATED APPLICATIONS

This application claims the benefit of priority to U.S. Provisional Application No. 63/049,866, filed on July 9, 2020.

### TECHNICAL FIELD

This disclosure relates generally to medical treatment systems and, more particularly, but not by way of limitation, to dressings, systems, and methods for treating a tissue site with reduced pressure.

### BACKGROUND

Clinical studies and practice have shown that reducing pressure in proximity to a tissue site can augment and accelerate growth of new tissue at the tissue site. The applications of this phenomenon are numerous, but have proven particularly advantageous for treating wounds. Regardless of the etiology of a wound, whether trauma, surgery, or another cause, proper care of a wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," and "vacuum-assisted closure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

While the clinical benefits of negative-pressure therapy are widely known, the cost and complexity of negative-pressure therapy can be a limiting factor in its application, and the development and operation of negative-pressure systems, components, and processes continues to present significant challenges to manufacturers, healthcare providers, and patients.

US 2019/0307935 A1 describes a negative wound therapy system comprising a foam manifold pad in which the foam is felted and comprises ovoidal pores.

### SUMMARY

Shortcomings with certain aspects of tissue treatment dressings, systems, and methods are addressed as shown and described in a variety of illustrative, non-limiting example embodiments herein.

The present invention relates to a foam manifold pad, as defined in claim 1. Preferred embodiments are defined in dependent claims 2-7.

For example, in some embodiments of the present disclosure the dressing may comprise a manifold pad which may be configured to contract laterally under applied negative pressure, which may provide lateral contractive force to a tissue site. For example, the manifold pad may comprise foam that is felted to create ovoidal pores or cells (e.g. with a major axis longer than its minor axis). In some embodiments, the ovoidal pores may be oriented so that the minor axis of the ovoidal pores is oriented parallel to the surface of the manifold pad (e.g. perpendicular to the thickness of the manifold pad) and the major axis of the ovoidal pores is oriented parallel to the thickness (e.g. perpendicular to the surface of the manifold pad). This ovoidal pore configuration may result in a manifold pad that contracts more radially or laterally upon application of negative pressure than in thickness, which may help draw incision edges closed.

More generally, some embodiments of the present disclosure may relate to a foam manifold pad for providing negative-pressure therapy to a tissue site, which may comprise: a first surface; a second surface; and a thickness of foam extending between the first surface and the second surface; wherein the foam of the manifold pad may comprise a plurality of ovoidal pores, and the manifold pad and/or the ovoidal pores may be configured to contract in a plane substantially or approximately parallel to the first surface and/or approximately perpendicular to the thickness upon application of negative pressure. In some embodiments, the manifold pad and/or ovoidal pores may be configured to preferentially contract in a plane substantially parallel to the first surface. In some embodiments, the manifold pad may be configured to resist contraction in thickness, for example contracting less in thickness than laterally or radially under negative pressure. In some embodiments, the manifold pad may be configured to contract more in a plane substantially perpendicular to the thickness than in the thickness, upon application of negative pressure. In some embodiments, the manifold pad may be configured to be substantially free of contraction in thickness in a direction extending from the first surface to the second surface under negative pressure. In some embodiments, the manifold pad may be configured so that there may be no significant contraction of the thickness of the manifold pad under applied negative pressure. In some embodiments, the foam may comprise open-cell foam. For example, the open-cell foam of the manifold pad may have a cell-structure of open cells or pores, and the ovoidal pores (e.g. open cells) may be formed by the cell-structure.

In some embodiments, the ovoidal pores may each comprise a major axis and a minor axis; the major axis may be oriented substantially perpendicular to the first surface; the minor axis may be oriented substantially parallel to the first surface; for each ovoidal pore, the major axis may be substantially perpendicular to the minor axis; and for each ovoidal pore, a length along the major axis may be longer than a length along the minor axis. In some embodiments, the ovoidal pores may be configured so that, upon application of negative pressure, the ovoidal pores contract more in the direction of the minor axis than in the direction of the major axis. In some embodiments, the foam of the manifold pad may comprise felted foam with an axis (e.g. direction) of compression, and the minor axis of the ovoidal pores may be substantially parallel to the axis of compression. For example, the felted foam may be compressed (e.g. felted) to have a firmness factor of 2-7. In some embodiments, the ratio of contraction of the minor axis of the ovoidal pores to contraction of the major axis, under applied negative pressure, may be greater than 1.

Some other examples of manifold pad embodiments may comprise: a first surface; a second surface; and a thickness of foam extending between the first surface and the second surface; wherein the foam of the manifold pad may comprise a cell-structure having a plurality of ellipsoidal or elongate cells. In some embodiments, the manifold pad may be configured to preferentially contract in a plane approximately perpendicular to the thickness upon application of negative pressure. In some embodiments, each of the ellipsoidal cells may comprise a major axis and a minor axis; the major axis of the ellipsoidal cells may be oriented approximately parallel to the thickness of the manifold pad; the minor axis of the ellipsoidal cells may be oriented approximately perpendicular to the thickness of the manifold pad; and the ellipsoidal cells may be configured to preferentially contract in a plane approximately perpendicular to the thickness upon application of negative pressure. In some embodiments, the major axis of the ellipsoidal cells may be oriented to be no more than 44 degrees from parallel to the thickness. In some embodiments, the major axis of the ellipsoidal cells may be oriented to be no more than 25 degrees or no more than 20 degrees from parallel to the thickness. In some embodiments, the foam of the manifold pad may comprise felted foam having the cell-structure with the plurality of ellipsoidal pores; and the felted foam of the manifold pad may be configured to be more resistant to contraction of the thickness, under applied negative pressure, than similar unfelted foam; to provide greater lateral contraction, under applied negative pressure, than similar unfelted foam; and/or to transmit a higher lateral closure force, under applied negative pressure, as compared to similar unfelted foam.

Some dressing embodiments of the present disclosure, for providing negative-pressure therapy to a tissue site, may comprise a foam manifold pad as described herein. In some embodiments, the dressing may further comprise a treatment device, which may be configured as a viscera protection layer to provide separation between the abdominal wall and the viscera and to protect abdominal contents. In some embodiments, the treatment device may have a film layer and may be configured to channel fluid radially under applied negative pressure. In some embodiments, the treatment device may comprise a central hub and a plurality of elongate members extending outward from the central hub. In some embodiments, the treatment device may further comprise a treatment manifold, which may be attached to the film layer. In some embodiments, the treatment manifold may comprise open-cell foam. In some embodiments, the treatment manifold may be enveloped or encapsulated by the film layer, which may be a non-adherent drape with a plurality of fenestrations. In some embodiments, the treatment manifold may comprise a central connection manifold member and a plurality of leg manifold members extending outward from the central connection manifold member. Some embodiments may further comprise a sealing member configured to cover the manifold pad and the treatment device and to provide a pneumatic seal relative to the tissue site. In some embodiments, the sealing member may be configured to be disposed over the treatment device and manifold pad to create a sealed space on the tissue site. In some embodiments, the manifold pad may be configured so that the first surface faces towards and/or contacts the sealing member and the second surface faces towards and/or contacts the treatment device. In some embodiments, the manifold pad may be configured to distribute applied negative pressure to the treatment device (and thereby to the tissue site).

System embodiments of the present disclosure may comprise a dressing as described herein; and a negative-pressure source fluidly coupled to the dressing. In some embodiments, the negative-pressure source may fluidly couple to the sealed space formed by the dressing. For example, the negative-pressure source may fluidly communicate with the manifold pad through the sealing member, and the applied negative pressure may be distributed through the manifold pad, through the treatment device, to the tissue site. In some embodiments, the dressing may be used on a tissue site with an incision, and the application of negative pressure to the manifold pad may draw the incision edges closer together.

Method embodiments of the present disclosure, for forming a manifold pad or dressing, are also disclosed, and may comprise: providing a blank of foam having a plurality of pores; altering the foam blank to form foam, wherein altered pores of the foam each are ovoidal with a major axis and a minor axis; and shaping the foam to form a manifold pad having a first surface, a second surface, and a thickness of foam extending between the first surface and the second surface. In some embodiments, the minor axis may be substantially parallel to the first surface and/or substantially perpendicular to the thickness. In some embodiments, altering the foam blank may comprise compressing the foam blank along an axis (e.g. direction) of compression to permanently deform the plurality of pores to form the foam with ovoidal pores having the major axis substantially perpendicular to the axis of compression and the minor axis substantially parallel to the axis of compression. In some embodiments, the foam blank may be heated as it is compressed. In some embodiments, altering the foam blank may comprise felting the foam blank to a firmness factor of 2-7 along the axis of compression so that the foam is formed with ovoidal pores having the major axis substantially perpendicular to the axis of compression and the minor axis substantially parallel to the axis of compression. In some embodiments, shaping the foam may comprise cutting the foam perpendicular to the axis of compression. In some embodiments, shaping the foam may comprise cutting the foam to form the first surface substantially parallel to the minor axis and the major axis substantially perpendicular to the first surface. In some embodiments, shaping the foam may comprise rotating the foam 90 degrees (e.g. rotating the axis of compression 90 degrees after compression), and then cutting the foam through the thickness (e.g. perpendicular to the axis of compression).

Methods of treating a tissue site are also disclosed, and may comprise: applying a treatment device as described herein, to the tissue site; applying a manifold pad, similar to those described herein, over the treatment device; and applying a sealing member over the manifold pad to form a sealed space on the tissue site. In some embodiments, the manifold pad may be applied with the first surface substantially parallel to a portion of the tissue site underlying the manifold pad, with the ovoidal pores oriented with the major axis substantially perpendicular to the first surface, with the minor axis substantially parallel to the first surface, with the minor axis substantially parallel to the portion of the tissue site underlying the manifold pad, and/or with the minor axis substantially parallel to a portion of the sealing member disposed over the manifold pad. Some embodiments may further comprise applying negative pressure to the manifold pad, and responsive to application of the negative pressure, contracting the manifold pad in a plane substantially parallel to the first surface without significantly drawing down (e.g. reduction) of the thickness. In some embodiments, applying negative pressure may comprise fluidly coupling a negative-pressure source to the manifold pad through the sealing member.

Other aspects, features, and advantages of the illustrative example embodiments will become apparent with reference to the drawings and detailed description that follow.

### BRIEF DESCRIPTION OF THE DRAWINGS

FIGURE 1A is a schematic diagram, with a portion in cross-section, of an illustrative example embodiment of an open-cavity, reduced-pressure treatment device and system;
FIGURE 1B is a schematic cross-section of a portion of the example treatment device of FIGURE 1A;
FIGURE 1C is a schematic cross-section of a portion of the example treatment device of FIGURE 1A taken along line 1C-1C;
FIGURE 1D is a schematic cross-section of a portion of the example system of FIGURE 1A;
FIGURE 2 is a schematic, perspective view of the example open-cavity, reduced-pressure treatment device of FIGURES 1A-1D;
FIGURE 3A is a schematic, plan view of another illustrative example embodiment of an open-cavity, reduced-pressure treatment device;
FIGURE 3B is a schematic, plan view of a portion of the example treatment device of FIGURE 3A;
FIGURE 3C is a schematic cross-section of a portion of the example treatment device of FIGURE 3B taken along line 3C-3C;
FIGURE 4 is a perspective view of an illustrative example embodiment of a manifold pad according to this disclosure;
FIGURE 5 is a schematic diagram of a portion of an embodiment of a dressing of FIGURE 1A, illustrating additional details regarding an exemplary embodiment of the manifold pad;
FIGURE 6 is a detail view of an enlarged portion of FIGURE 5, illustrating in more detail the shape and orientation of the pores/cells of the foam of the manifold pad; and
FIGURE 7 is a schematic diagram illustrating compression and/or felting of a foam blank to form the foam of an exemplary manifold pad similar to FIGURES 5-6.

### DETAILED DESCRIPTION OF ILLUSTRATIVE EMBODIMENTS

The following description of example embodiments enables a person skilled in the art to make and use the subject matter set forth in the appended claims. Certain details already known in the art may be omitted. Therefore, the following detailed description is illustrative and non-limiting.

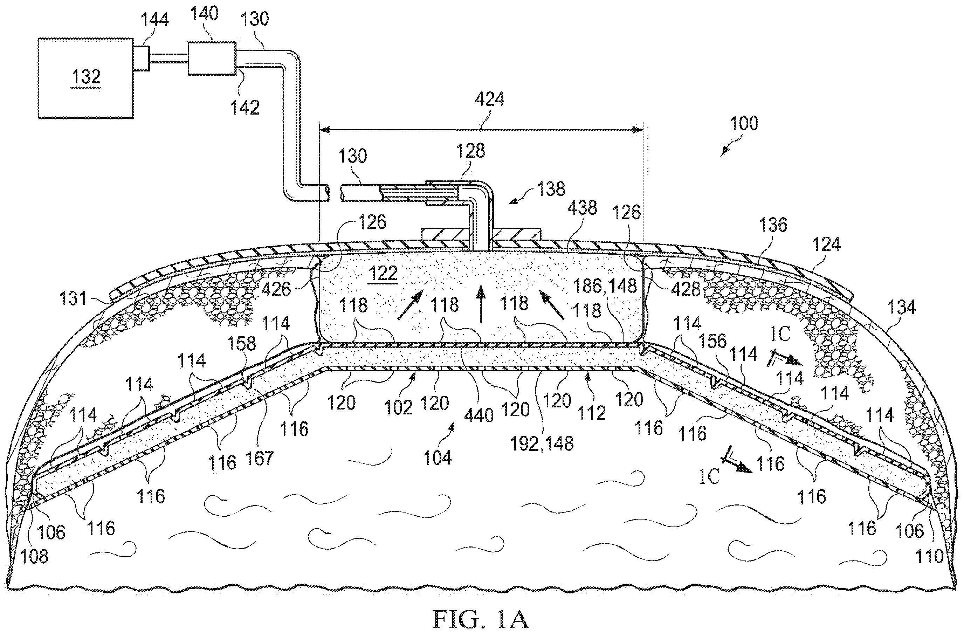

Referring to FIGURES 1A-1D, an illustrative embodiment of an open-cavity, reduced-pressure system 100 and a treatment device 102 is presented. The open-cavity reduced-pressure system 100 and the treatment device 102 are for treating a tissue site 104 of a patient. The tissue site 104 may be the bodily tissue of any human, animal, or other organism, including bone tissue, adipose tissue, muscle tissue, dermal tissue, tissue, connective tissue, cartilage, tendons, ligaments, or any other tissue. In this illustrative embodiment, the tissue site 104 includes tissue in a body cavity, and in particular the abdominal cavity, and includes the abdominal contents or tissue that is proximate the abdominal cavity. Treatment of the tissue site 104 may include removal of fluids, e.g., exudate or ascites, protection of the abdominal cavity, or reduced-pressure therapy.

As shown, the treatment device 102 is disposed within the abdominal cavity of the patient to treat the tissue site 104. The treatment device 102 of FIGURES 1A-D includes a plurality of encapsulated leg members 106 that are supported by the abdominal contents, which make up a surface on which the plurality of leg members 106 are placed. One or more of the plurality of encapsulated leg members 106 may be placed in or proximate to a first paracolic gutter 108, and one or more of the plurality of encapsulated leg members 106 may be placed in or proximate to a second paracolic gutter 110. The plurality of encapsulated leg members 106 is coupled to a central connection member 112, and there is fluid communication between the plurality of encapsulated leg members 106 and the central connection member 112. The plurality of encapsulated leg members 106 and/or the central connection member 112 may be formed with fenestrations 114, 116, 118, 120 that allow fluids in the abdominal cavity to pass through. The fenestrations 114, 116, 118, 120 may take any shape, e.g., circular apertures, rectangular openings, polygons, etc., but are presented in this illustrative embodiment as slits, or linear cuts. One or more fenestrations 114, 116, 118, 120 might be omitted in alternative embodiments.

A manifold pad 122, distributes reduced pressure to the treatment device 102. A sealing member 124 provides a pneumatic seal over body-cavity opening 126. One or more skin closure devices may be placed on a patient's epidermis 134. Reduced pressure is delivered to the manifold pad 122 through a reduced-pressure interface 128, which is coupled to a reduced-pressure delivery conduit 130. A reduced-pressure source 132 delivers reduced pressure to the reduced-pressure delivery conduit 130.

The reduced pressure may be applied to the tissue site 104 to help promote removal of ascites, exudates, or other fluids from the tissue site 104. In some instances, reduced pressure may be applied to stimulate the growth of additional tissue. In some instances, only fluid removal may be desired. In the case of a wound at the tissue site 104, the growth of granulation tissue, removal of exudates, or removal of bacteria may help to promote healing of the wound. In the situation of a non-wounded or non-defective tissue, reduced pressure may be used in some instances to promote the growth of tissue that may be harvested and transplanted to another tissue site.

As used herein, "reduced pressure" generally refers to a pressure less than the ambient pressure at a tissue site being subjected to treatment. Typically, this reduced pressure will be less than the atmospheric pressure. The reduced pressure may also be less than a hydrostatic pressure at a tissue site. Unless otherwise indicated, values of pressure stated herein are gauge pressures. While the amount and nature of reduced pressure applied to a tissue site will typically vary according to the application, the reduced pressure will typically be between -5 mm Hg and -500 mm Hg, and more typically in a therapeutic range between -100 mm Hg and -200 mm Hg. For example, therapeutic reduced pressure may be approximately -125 mm Hg in some applications. Reduced pressure may also be termed "negative pressure", and the terms may be used interchangeably.

The reduced pressure delivered may be constant, varied, patterned, or random, and may be delivered continuously or intermittently. Although the terms "vacuum" and "negative pressure" may be used to describe the pressure applied to the tissue site, the actual pressure applied to the tissue site may be more than the pressure normally associated with a complete vacuum. Consistent with the use herein, an increase in reduced pressure corresponds to a reduction in pressure (more negative relative to ambient pressure) and a decrease in reduced pressure corresponds to an increase in pressure (less negative relative to ambient pressure).

Reduced pressure may initially generate fluid flow in the manifold pad 122, the reduced-pressure conduit 130, and proximate the tissue site 104. As the hydrostatic pressure around the tissue site 104 approaches the desired reduced pressure, the flow may subside, and the reduced pressure may be maintained.

The manifold pad 122 is proximate the central connection member 112. The manifold pad 122 may take many forms. The term "manifold" as used herein generally refers to a substance or structure that is provided to assist in applying reduced pressure to, delivering fluids to, or removing fluids from the tissue site 104. The manifold pad 122 typically includes a plurality of flow channels or pathways that distribute the fluids provided to and removed from the tissue site 104 around the manifold pad 122 and through the central connection member 112. In one illustrative embodiment, the flow channels or pathways are interconnected to improve distribution of fluids provided or removed from the tissue site 104. The manifold pad 122 may be a biocompatible material that is capable of being placed in contact with the tissue site 104 and distributing reduced pressure to the tissue site 104.

Examples of suitable materials for the manifold pad 122 may include, without limitation, devices that have structural elements arranged to form flow channels, cellular foam, such as open-cell foam, porous tissue collections, liquids, gels and foams that include or cure to include flow channels. The manifold pad 122 may be porous and may be made from foam, gauze, felted mat, silicone, polyvinyl alcohol, or any other material suited to a particular biological application. In one embodiment, the manifold pad 122 is a porous foam and includes a plurality of interconnected cells or pores that act as pathways or flow channels. The porous foam may be a polyurethane or polyether, open-cell, reticulated foam, such as GRANUFOAM^{™} material available from Kinetic Concepts, Incorporated in San Antonio, Texas. Other embodiments might include "closed cells." These closed-cell portions of the manifold may contain a plurality of cells, the majority of which are not fluidly connected to adjacent cells. The closed cells may be selectively disposed in the manifold pad 122 to prevent transmission of fluids through perimeter surfaces of the manifold pad 122. In some situations, the manifold pad 122 may also be used to distribute fluids such as medications, antibacterials, growth factors, and various solutions to the tissue site 104. Other layers may be included in or on the manifold pad 122, such as absorptive materials, wicking materials, hydrophobic materials, and hydrophilic materials.

A material with a higher or lower density than GRANUFOAM^{™} material may be desirable depending on the application. Among the many possible materials, the following may be used: GRANUFOAM^{™} material, FOAMEX^{™} technical foam, a molded bed of nails structures, a patterned grid material such as those manufactured by Sercol Industrial Fabrics, 3D textiles such as those manufactured by Baltex of Derby, United Kingdom, a gauze, a flexible channel-containing member, a graft, or similar materials. In some instances, ionic silver may be added to the manifold pad 122 by, for example, a micro bonding process. Other substances, such as anti-microbial agents, may be added to the manifold pad 122 as well.

The sealing member 124 is configured to be placed over the body-cavity opening 126 and may be formed from any material capable of providing a pneumatic seal or fluid seal adequate for the open-cavity, reduced-pressure system 100 to hold a reduced pressure at the tissue site 104. The sealing member 124 may be a cover that is used to secure the manifold pad 122 on the central connection member 112. The sealing member 124 may be impermeable or semi-permeable. The sealing member 124 is capable of maintaining reduced pressure at the tissue site 104 after installation of the sealing member 124 over the body-cavity opening 126. The sealing member 124 may be a flexible over-drape or film formed from a silicone-based compound, acrylic, hydrogel or hydrogel-forming material, or any other biocompatible material that includes the impermeability or permeability characteristics as desired for applying reduced pressure to the tissue site 104.

More specifically, the sealing member 124 may comprise, for example, one or more of the following materials: hydrophilic polyurethane; cellulosics; hydrophilic polyamides; polyvinyl alcohol; polyvinyl pyrrolidone; hydrophilic acrylics; hydrophilic silicone elastomers; an INSPIRE 2301 material from Expopack Advanced Coatings of Wrexham, United Kingdom having, for example, an MVTR (inverted cup technique) of 14400 g/m2/24 hours and a thickness of about 30 microns; a thin, uncoated polymer drape; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; polyurethane (PU); EVA film; co-polyester; silicones; a silicone drape; a TEGADERM^{™} drape available from 3M; a polyurethane (PU) drape such as one available from Avery Dennison Corporation of Pasadena, California; polyether block polyamide copolymer (PEBAX), for example, from Arkema, France; EXPOPACK 2327; or other appropriate material.

Further, the sealing member 124 may be vapor permeable and/or liquid impermeable, thereby allowing vapor and inhibiting liquids from exiting a sealed space between the sealing member 124 and the tissue site 104. In some embodiments, the sealing member 124 may be a flexible, breathable film, membrane, or sheet having a high moisture vapor transfer rate (MVTR) of, for example, at least about 250g/m² per 24 hours. In other embodiments, a low or no vapor transfer drape might be used. The sealing member 124 may comprise a range of medically suitable films having a thickness up to about 50 microns (µm).

The sealing member 124 may further include an attachment means 131 to secure the sealing member 124 to the patient's epidermis 134. The attachment means 131 may take many forms; for example, an adhesive layer 136 may be positioned along a perimeter of the sealing member 124 or any portion of the sealing member 124 to provide, directly or indirectly, the pneumatic seal with the patient's epidermis 134. The adhesive layer 136 might also be pre-applied to the sealing member 124 and covered with a releasable backing, or member (not shown), that is removed at the time of application.

The reduced-pressure interface 128 may be, as one example, a port or connector 138, which permits the passage of fluid from the manifold pad 122 to the reduced-pressure delivery conduit 130 and vice versa. For example, fluid collected from the tissue site 104 using the manifold pad 122 and the treatment device 102 may enter the reduced-pressure delivery conduit 130 via the connector 138. In another embodiment, the open-cavity, reduced-pressure system 100 may omit the connector 138 and the reduced-pressure delivery conduit 130 may be inserted directly into the sealing member 124 and into the manifold pad 122. The reduced-pressure delivery conduit 130 may be a medical conduit or tubing or any other means for transportating a reduced pressure and fluid. The reduced-pressure delivery conduit 130 may be a multi-lumen member for readily delivering reduced pressure and removing fluids. In one embodiment, the reduced-pressure delivery conduit 130 is a two-lumen conduit with one lumen for reduced pressure and liquid transport and one lumen for communicating pressure to a pressure sensor.

Reduced pressure is generated and supplied to the reduced-pressure delivery conduit 130 by the reduced-pressure source 132. A wide range of reduced pressures may be generated or supplied by the reduced-pressure source 132. In one embodiment, the range may include -50 to -300 mm Hg, and in another embodiment, the range may include -100 mm Hg to -200 mm Hg. In one illustrative embodiment, the reduced-pressure source 132 includes preset selectors for -100 mm Hg, - 125 mm Hg, and -150 mm Hg. 1 mm Hg = 133.322 Pa. The reduced-pressure source 132 may also include a number of alarms, such as a blockage alarm, a leakage alarm, or a battery-low alarm.

The reduced-pressure source 132 may be any suitable device for providing reduced pressure, such as, for example, a vacuum pump, wall suction, hand pump, manual pump, electronic pump, micro-pump, piezoelectric pump, diaphragm pump, a portable source, wall source, a unit for abdominal cavities, or other source. The reduced-pressure source 132 may include control circuitry and sensors, such as a pressure sensor, that may be configured to monitor reduced pressure at the tissue site 104. The reduced-pressure source 132 may also be configured to control the amount of reduced pressure from the reduced-pressure source 132 being applied to the tissue site 104 according to a user input and a reduced-pressure feedback signal received from the tissue site 104. The reduced-pressure source 132 may selectively deliver a constant pressure, varied pressure, intermittent pressure, or continuous pressure. The fluid removed from the cavity through the reduced-pressure delivery conduit 130 could be as much as 5 liters or more per day.

A number of different devices, e.g., device 140, may be added to a medial portion 142 of the reduced-pressure delivery conduit 130. For example, the device 140 might be a fluid reservoir, or canister collection member, a pressure-feedback device, a volume detection system, a blood detection system, an infection detection system, a filter, a port with a filter, a flow monitoring system, a temperature monitoring system, etc. Multiple devices 140 might be included. Some of these devices, e.g., the fluid collection member, may be formed integral to the reduced-pressure source 132. For example, a reduced-pressure port 144 on the reduced-pressure source 132 may include a filter member (not shown) that includes one or more filters and may include a hydrophobic filter that prevents liquid from entering an interior space of the reduced-pressure source 132.

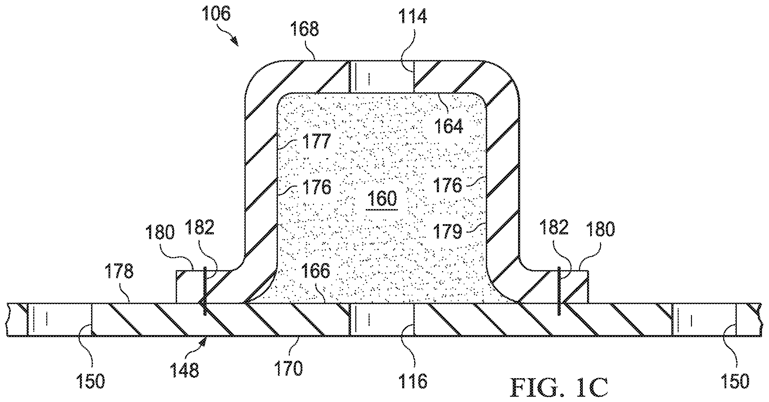

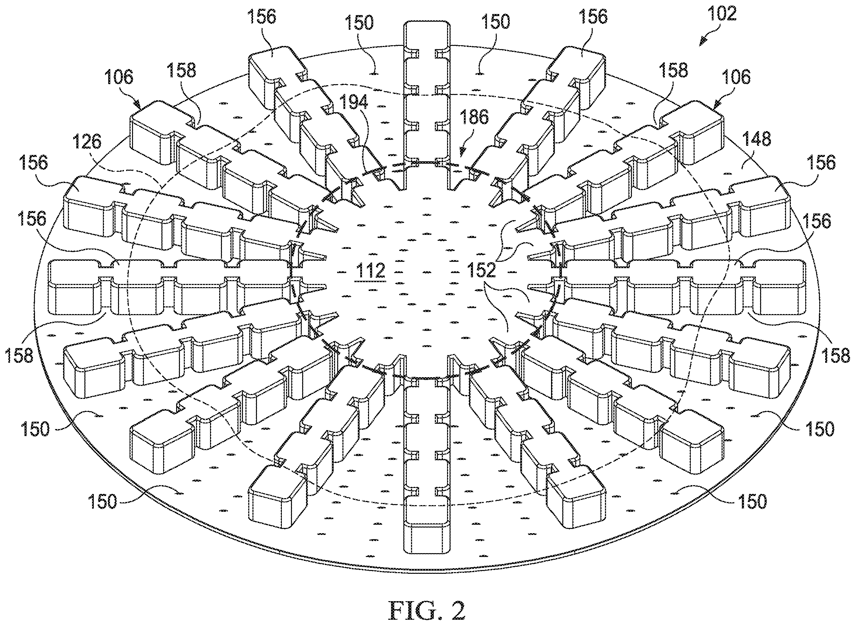

Referring now to FIGURES 1A, 1C, and 2, the treatment device 102 can include a non-adherent drape 148. The non-adherent drape 148 may be formed of a non-adherent material that inhibits tissue adhesion to the non-adherent drape 148. In one embodiment, the non-adherent drape 148 is formed from a breathable polyurethane film. The non-adherent drape 148 can include a plurality of openings, apertures, or fenestrations 150. The fenestrations can take a variety of shapes, such as circular openings, rectangular openings, polygon-shaped openings, etc., but are shown in FIGURE 2 as slits, or linear cuts. Depending on the particular application of device 102, the desired fluid flow and/or pressure delivery, or other system parameters, the fenestrations can have different sizes.

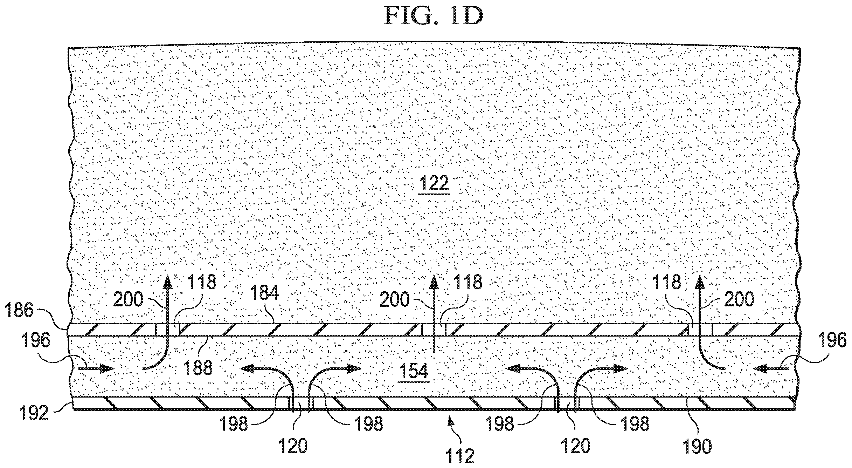

Referring to FIGURES 1A, 1D, and 2, the treatment device 102 includes the central connection member 112 to which the plurality of encapsulated leg members 106 are coupled. The central connection member 112 includes a connection manifold member 154 that is encapsulated by a first connection encapsulation member 186, which may be referred to as a first non-adherent drape 186, and a second connection encapsulation member 192, which may be referred to as a second non-adherent drape 192. A portion of the central connection member 112 can fluidly couple at leg coupling areas 152 to permit fluid communication between the central connection member 112 and the plurality of encapsulated leg members 106. The first and the second connection encapsulation member or non-adherent drape 186, 192 may be defined by a single piece of material or, as illustrated, more than one sheet of material. For example, the non-adherent drape 148 may be used as a single sheet or may include the first non-adherent drape 186 and the second non-adherent drape 192.

The central connection member 112, as discussed above, can fluidly communicate with manifold pad 122. In one aspect, fenestrations 118, similar to the fenestrations discussed above, can permit fluid communication. Additionally, or alternatively, a portion or portions of the first connection encapsulation member or non-adherent drape 186 can be exposed to manifold pad 122.

Referring again to FIGURES 1A-1D, each of the plurality of encapsulated leg members 106 can include a leg manifold member 160, which may be a single manifold member that runs between leg modules 156 and/or central connection member 112, or individual manifold components. The leg manifold member 160 is disposed within an interior portion 162 of each of the encapsulated leg members 106. Each leg manifold member 160 has a first side 164 and a second, inward-facing (patient-facing) side 166.

In some embodiments, the connection manifold member 154 and one or more of the leg manifold members 160 extending from the connection manifold member 154 may form a treatment manifold 167. The treatment manifold 167 including the leg manifold members 160 and the connection manifold member 154 may comprise or be formed of a foam or a manifold material similar or analogous to the manifold materials described herein for the manifold pad 122.

The non-adherent drape 148 may surround the treatment manifold 167. For example, the non-adherent drape 148 may be coupled around the leg manifold members 160 and the connection manifold member 154 to provide the plurality of encapsulated leg members 106 in fluid communication with the central connection member 112. In some embodiments, the non-adherent drape 148 may include the first non-adherent drape 186 and the second non-adherent drape 192 and the treatment manifold 167 may be positioned as a layer between the first non-adherent drape 186 and the second non-adherent drape 192. The first non-adherent drape 186 may be coupled to the second non-adherent drape 192 to provide the plurality of encapsulated leg members 106 in fluid communication with the central connection member 112.

In one embodiment, one or more of the plurality of leg manifold members 160 can have different material properties or structures. For example, different flow rates may be desired in different encapsulated leg members 106. In one aspect, different manifold materials or manifold properties, different manifold sizes, manifold compression, the use flow restricting material structures, and/or or valves can provide different flow rates of fluid through the encapsulated leg members and/or central connection member.

In one aspect, a first leg encapsulating member 168, which can be formed with fenestrations 114, is disposed on the first side 164 of the leg manifold member 160. A second leg encapsulating member 170, which can include fenestrations 116, is disposed on the second, inward-facing side 166 of the leg manifold member 160. The first leg encapsulating member 168 and the second leg encapsulating member 170 may be a portion of the non-adherent drape 148. In some embodiments, the first leg encapsulating member 168 may be a portion of the first non-adherent drape 186 and the second leg encapsulating member 170 may be a portion of the second non-adherent drape 192. In some embodiments, the encapsulated leg members 106 can be mated with one another, for example, via the drape 148. In some embodiments, the encapsulated leg members 106 can be independently movable with respect to one another with the exception of their proximal end adjacent to the central connection member 112. For example, the encapsulated leg members 106 need not be connected to one another. In another embodiment, a portion of the material connecting the encapsulated leg members 106, *e.g.*, the non-adherent drape 148 between adjacent encapsulated leg members 106, is expandable (*e.g*., a stretchable, flexible, deformable, and/or elastic material) and permits movement of individual encapsulated leg members 106 relative to one another.

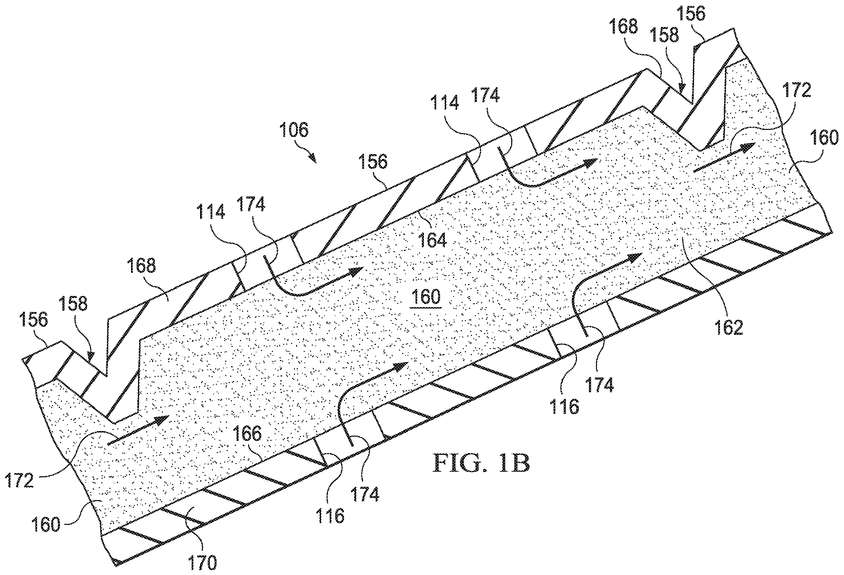

As shown in the longitudinal cross section of FIGURE 1B by arrows 172, fluid can flow from leg modules 156 towards the central connection member 112. As shown by arrows 174, the fluid is able to enter fenestrations 114 and 116 and flow into the leg manifold member 160 and then flow toward the central connection member 112 as represented by arrows 172.

In plan view, the encapsulated leg members 106 may take a number of different shapes, such as elongate shapes, rectangular, elliptical, etc. In one aspect, the encapsulated leg members 106 may include leg modules 156. Adjacent leg modules 156 are fluidly coupled to each other and have a manipulation zone 158 between them. In one aspect, the manipulation zone includes a weakened or perforated area to facilitate sizing of the device. For example, a clinician can cut through a leg module to size the device. By pulling on the partially cut leg module the manifold can be torn away at the next manipulation zone. In one aspect, the recessed shape of the manipulation zone 158 can inhibit accidental removal of additional leg modules. Additionally, or alternatively, the outer portion of the leg modules can be fixed to the device to inhibit unwanted manifold removal.

The encapsulated leg members 106 may also have various dimensions. If the longer dimension, e.g., lengthwise or longitudinal dimension, of the encapsulated leg 106 is _{L1} and the width is _{W1}, then the aspect ratio is given by _{L1}/_{W1}. The aspect ratio may be 8.0, 7.0, 6.0, 5.0, 4.0, 3.0, 2.0 or any number in between. Moreover, other aspect ratios are possible. Generally, the width _{W1} of the encapsulated leg member will be greater than a width _{W2} of the central connection member 112, *i.e*., _{W2} > _{W1}. For example, in one illustrative embodiment, the encapsulated leg members 106 are approximately 270 mm long, 60 mm wide (_{W1}), and 10 mm thick, and the central connection member has a width parallel to the first width (_{W1}) of about 130 mm (_{W2}). Thus, in that illustrative example, the aspect ratio of the encapsulated leg 106 is approximately (270/60) or 4.5. In this same illustrative embodiment, the manipulation zones 158 have a width of about 10 mm.

Referring to FIGURE 1C, a lateral cross section of a portion of the encapsulated leg member 106 is presented. As before, it can be seen that the first side 164 of the leg manifold member 160 is covered with the first leg encapsulating member 168, and that the second, inward-facing side 166 of the leg manifold member 160 is covered by the second leg encapsulating member 170, which in this instance is a portion of the non-adherent drape 148. Thus, in this illustrative embodiment, the fenestrations 116 may be some of the plurality of fenestrations 150 in the non-adherent drape 148. In this illustrative embodiment, peripheral edges 176 of the leg manifold member 160 are also covered by a portion of the first leg encapsulating member 168. The peripheral edges 176 include a first lateral edge 177 and a second lateral edge 179. The first leg encapsulating member 168 covers the first side 164 and the peripheral edges 176 and extends onto a first surface 178 of the non-adherent drape 148 and forms extensions 180. The extensions 180 have been coupled to the second leg encapsulating member 170 by welds 182. The first leg encapsulating member 168 may, however, be coupled to the second leg encapsulating member 170 using any known technique, including welding (e.g., ultrasonic or RF welding), bonding, adhesives, cements, etc.

In some embodiments, at least a portion of a longitudinal length of at least one of the encapsulated leg members 106 is configured to contact the tissue site 104. Further, in some embodiments, the fenestrations 116 may be positioned along the longitudinal length of at least one of the encapsulated leg members 160.

Referring again to FIGURE 1D and FIGURE 2, the central connection member 112 includes the connection manifold member 154 that is encapsulated within the first connection encapsulation member 186, which has fenestrations 118. The first connection encapsulation member 186 is disposed on a first side 188 of the connection manifold member 154. The second connection encapsulation member 192 is disposed on a second, inward-facing side 190 of the connection manifold member 154. The second connection encapsulation member 192 is formed with fenestrations 120. The first connection encapsulation member 186 has a peripheral zone or edge 194 as shown in FIGURE 2. In a similar fashion, the second connection encapsulation member 192 has a peripheral zone or edge (not explicitly shown) that lines up with the peripheral edge 194. The peripheral edge 194 of the first connection encapsulation member 186 is coupled to peripheral edge of the second connection encapsulation member 192, except at the leg coupling areas 152 in order to allow fluid within the plurality of encapsulated leg members 106 to flow into the connection manifold member 154 as suggested by arrows 196 in FIGURE 1D. Fluid may also enter directly into the connection manifold member 154 by flowing through fenestrations 120 as suggested by arrows 198. The manifold pad 122 is disposed proximate to the first connection encapsulation member 186, and when a reduced pressure is applied to the manifold pad 122, the reduced pressure causes fluid to flow from the connection manifold member 154 through fenestrations 118 and into the manifold pad 122 as suggested by arrows 200. The fluid continues to flow in the direction of the reduced-pressure interface 128 through which the fluid is removed to the reduced-pressure delivery conduit 130.

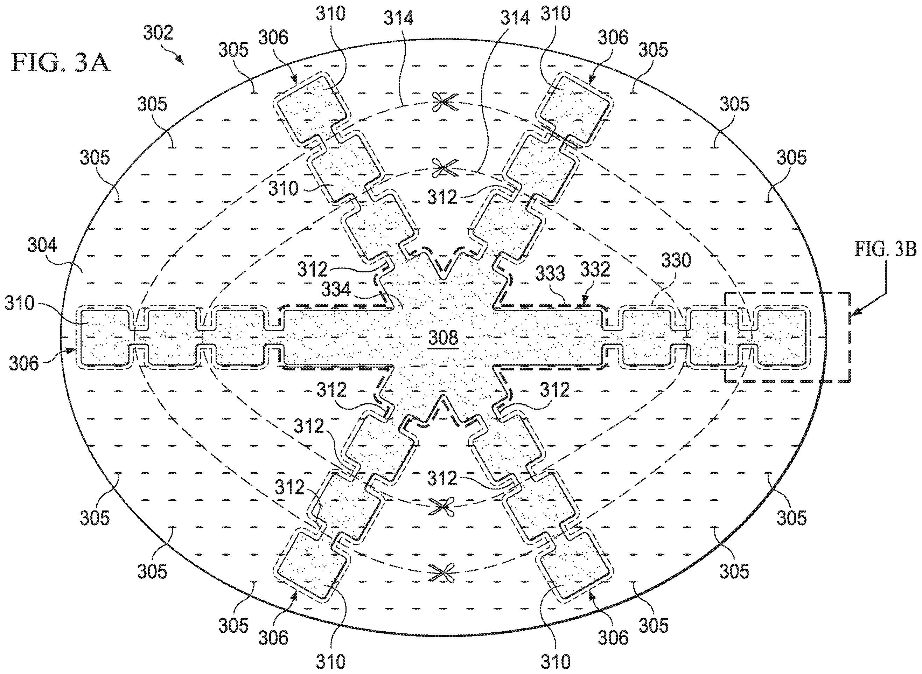

Referring to FIGURES 1A-1D and 2, in operation, the illustrative open-cavity, reduced-pressure system 100 may be used by first sizing the treatment device 102 as will be explained further below in connection with FIGURE 3A. The non-adherent drape 148 with the plurality of encapsulated leg members 106 is disposed within the abdominal cavity through the body-cavity opening 126 and is distributed against the abdominal contents; this may include placing at least one encapsulated leg member 106 in or proximate the first paracolic gutter 108, the second paracolic gutter 110, or behind the liver, etc. Once the treatment device 102 has been distributed, the manifold pad 122 is placed adjacent a first side 184 of the first connection encapsulation member 186. The sealing member 124 may then be applied over the body-cavity opening 126 to provide a pneumatic seal over the body-cavity opening 126.

In addition to the sealing member 124, the body-cavity opening 126 may be further closed or reinforced using mechanical closing means, e.g., staples or tension cords (such as Derma-close or ABRA surgical skin closure), or using a reduced-pressure closure system. The sealing member 124 may be applied in a number of ways, but according to one illustrative embodiment, the releasable backing member that is on the adhesive layer 136 of the sealing member 124 is removed and then the sealing member 124 is placed against the patient's epidermis 134 about the body-cavity opening 126. The reduced-pressure interface 128, such as port 138, is then coupled or attached to the sealing member 124 such that reduced pressure can be delivered by the interface 128, through the sealing member 124, and to the manifold pad 122 and the central connection member 154. The reduced-pressure delivery conduit 130 is fluidly coupled to the reduced-pressure interface 128 and to the reduced-pressure port 144 on the reduced-pressure source 132.

The reduced-pressure source 132 is activated and thereby provides reduced pressure into the reduced-pressure delivery conduit 130, which delivers the reduced pressure to the reduced-pressure interface 128 and into the manifold pad 122 and the central connection member 154. The manifold pad 122 distributes the reduced pressure and draws fluid through fenestrations 118 from the connection manifold member 154. The connection manifold member 154 draws fluid from the abdominal cavity through fenestrations 120 and pulls fluid from the plurality of encapsulated leg members 106 as suggested by arrows 196. Fluid from the abdominal cavity flows into the plurality of encapsulated leg members 106 through fenestrations 114 on the first leg encapsulating member 168 and through fenestrations 116 on the second leg encapsulating member 170 and then flows through the leg as suggested by arrows 172 towards the connection manifold member 154. The fluid then flows through the manifold pad 122, the reduced-pressure interface 128, and into the reduced-pressure delivery conduit 130.

Referring now to FIGURES 3A-3C, another illustrative embodiment of an open-cavity, reduced-pressure treatment device 302 is presented. The open-cavity, reduced-pressure treatment device 302 is analogous in most respects to the treatment device 102 of FIGURES 1A-1D. The open-cavity, reduced-pressure treatment device 302 has a non-adherent drape 304, a plurality of encapsulated leg members 306, and a central connection member 308. In this particular illustrative embodiment, the non-adherent drape 304 is formed generally with an oval or arcuate shape. The non-adherent drape 304 is formed with a plurality of fenestrations 305. The non-adherent drape 304 forms the second leg encapsulating member (see by analogy second leg encapsulating member 170 in FIGURE 1B) and the second connection encapsulation member (see by analogy 192 in FIGURE 1D). As such, the plurality of fenestrations 305 serves as flow channels for the plurality of encapsulated leg members 306 and the central connection member 308 on the second, inward-facing side. The non-adherent drape 304 could also be used on the first side of the plurality of encapsulated leg members 306 and the central connection member 308.

Each of the encapsulated leg members 306 may be formed with a plurality of leg modules 310 with manipulation zones 312 between the plurality of leg modules 310. As with the manipulation zone 158 in FIGURES 1A-D, the manipulation zones 312 may facilitate movement of the plurality of encapsulated leg members 306 within the body cavity and may provide an easier location at which to cut the plurality of encapsulated leg members 306 when the open-cavity, reduced-pressure treatment device 302 is being sized for a particular application. In this regard, visual indicia 314 may be added on the non-adherent drape 304 to help the healthcare provider know where to cut the non-adherent drape 304 for different sizes of application within the cavity. The visual indicia 314 may comprise cut lines, or graduations, that preferably run through the manipulation zones 312. In some embodiments, the manipulation zones 312 may provide a convenient and easy location for cutting the open-cavity, reduced-pressure treatment device 302. In other embodiments, the leg members 306 may be cut anywhere along their length. For example, in some embodiments the leg members 306 may be cut for sizing through the leg modules 310, and the foam of the leg member manifold may then be pulled inward with respect to the film or drape to prevent exposure to the tissue site.

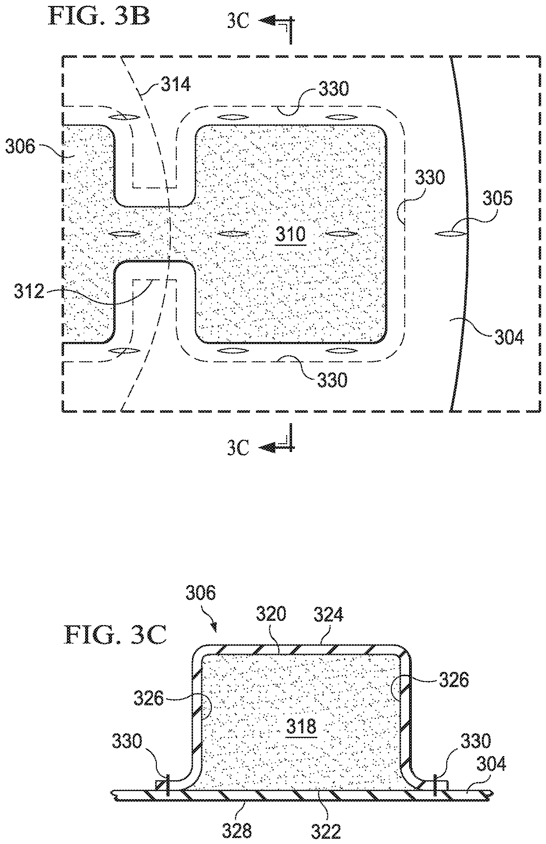

Referring to FIGURE 3C, a lateral cross section of a portion of an encapsulated leg member 306 is presented. The plurality of encapsulated leg members 306 are formed with a leg manifold member 318 having a first side 320 and a second, inward-facing (patient-facing) side 322. A first leg encapsulating member 324 covers the first side 320 of the leg manifold member 318 and covers a lateral zone or edge 326 of the leg manifold member 318. The second, inward-facing side 322 of the leg manifold member 318 is covered by a second leg encapsulating member 328, which in this embodiment is a portion of the non-adherent drape 304. The first leg encapsulating member 324 is coupled to the second leg encapsulating member 328 by any means known in the art, such as by welding (*e.g*., ultrasonic or RF), bonding, adhesives, cements, etc. In this illustrative embodiment, the first leg encapsulating member 324 and the second leg encapsulating member 328 are coupled by a weld 330. Referring to FIGURE 3B, the weld 330 is shown along the perimeter of the plurality of leg modules 310. In some embodiments, the weld 330 may be continuous along the perimeter of the leg module. In other embodiments the weld 330 may not be continuous. For example, the weld 330 may have small breaks along its length.

Referring again to FIGURE 3A, the central connection member 308 is formed analogously to the central connection member 112 in FIGURE 2. A first connection encapsulation member 334 and a second connection encapsulation member of the central connection member 308 are coupled along a peripheral edge 332 using a weld 333 or another coupling technique, such as those previously mentioned. The peripheral edge 332 is not sealed, however, proximate each of the encapsulated leg members 306 in order to provide a channel for fluid to flow from the plurality of encapsulated leg members 306 into the central connection member 308.

According to one illustrative approach to constructing the open-cavity, reduced-pressure treatment device 302, the non-adherent drape 304, which is already formed with the plurality of fenestrations 305 and that has visual indicia 314 is provided. The leg manifold member 318 is disposed adjacent the non-adherent drape 304. The central connection manifold 308 is disposed adjacent to the leg manifold member 318 or may be formed integral with leg manifold member 318. The first connection encapsulation member 334 is placed on the central connection member 308, and the first leg encapsulating member 324 is placed over the leg manifold member 318. The first connection encapsulation member 334 and the first leg encapsulating member 324 may be formed from an integral sheet. Next, the welds 330 and 333 are applied.

In an alternative embodiment for manufacturing an open-cavity, reduced-pressure treatment device, a first non-adherent drape 304, which includes fenestrations, may be provided and the leg manifold member 318 and the central connection manifold 308 disposed on the first non-adherent drape 304. A second non-adherent drape, which has fenestrations, is placed over the first non-adherent drape 304, the leg manifold member 318, and the central connection manifold 308. Next, a plurality of welds (*e.g*., thermal or RF or another coupling techniques used) are made, such as with the welds 330. The first non-adherent drape 304 and the second non-adherent drape may be cut to size before or after assembly. By using two drapes, the first non-adherent drape 304 and the second non-adherent drape may provide better distribution of reduced pressure and may ease the manufacturing process.

The fenestrations may be formed before or after assembly. The perimeter of the first non-adherent drape 304 and the second non-adherent drape may be welded. Other points may be welded between the drapes to form a single unit. In another alternative embodiment, the drapes may initially be placed and welded without fenestrations, and then fenestrations added to the drapes so that the fenestrations align. The fenestrations might also be formed using an electrical member that cuts and seals at the same time to form aligned, "button hole" fenestrations through the two drapes.

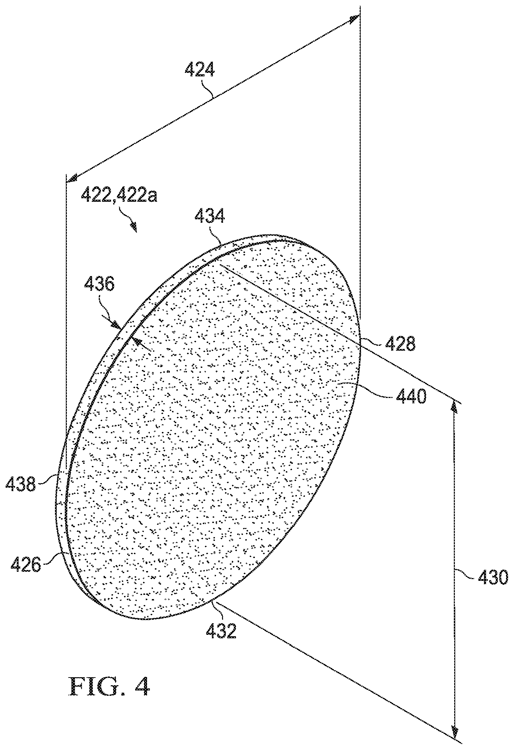

Referring to FIGURES 1A, 1D, and 4, in some example embodiments, the manifold pad 122 may be configured to distribute reduced pressure relative to the tissue 104 and other components of the system 100, such as, for example, the treatment device 102 or the treatment manifold 167. As such, the manifold pad 122 may be positioned proximate to and in fluid communication with the treatment device 102 or the treatment manifold 167. In some examples, the manifold pad 122 may be in fluid communication with the treatment manifold 167 through the non-adherent drape 148, or a portion of the non-adherent drape 148, such as the first non-adherent drape 186. Further, in some examples, the manifold pad 122 may be configured to be positioned in direct contact with the non-adherent drape 148 or the first non-adherent drape 186. Further, in some examples, the manifold pad 122 may be positioned proximate to and in fluid communication with the connection manifold member 154 or the central connection member 112. The sealing member 124 may be configured to cover the treatment manifold 167 and the manifold pad 122 at the tissue site 104.

Referring to FIGURES 1A and 4, in some example embodiments, the manifold pad 122 may include a lateral width 424 extending from a first edge 426 to an opposite second edge 428 of the manifold pad 122. The lateral width 424 may be configured to extend across the body-cavity opening 126 at the tissue site 104. Further, the manifold pad 122 may include a longitudinal length 430 extending from a first end 432 to an opposite second end 434 of the manifold pad 122. In some embodiments, the longitudinal length 430 may be greater than the lateral width 424 and perpendicular to the lateral width 424 of the manifold pad 122. The longitudinal length 430 may be configured to be positioned along a closure line that may be formed by opposing sides of the body-cavity opening 126, such as, for example, an incision, wound edges, or a line of staples or sutures, etc. In some embodiments, the incision or body cavity opening may be formed by an open wound having wound edges. In some embodiments, the wound edges of the body-cavity opening 126 (which may be an abdominal cavity) may extend longitudinally. Further, the manifold pad 122 may include a thickness 436 extending from a first side or surface 438 to an opposite second side or surface 440 of the manifold pad 122. The thickness 436 of the manifold pad 122 may be perpendicular to both the lateral width 424 and the longitudinal length 430 of the manifold pad 122. In some embodiments, the term perpendicular may include orthogonal. In some embodiments, the thickness 436 of the manifold pad 122 may range from about 5-25 mm, about 8-25 mm, about 8-16 mm, or about 5-16 mm. The first surface 438 of the manifold pad 122 may be configured to face outward from or away from the tissue site 104 and, for example, the treatment device 102, the treatment manifold 167, or portions thereof. In some embodiments, the first surface may be configured to face towards the sealing member 124. Further, the second surface 440 of the manifold pad 122 may be configured to face the tissue site 104 and, for example, the treatment device 102, the treatment manifold 167, or portions thereof. The first surface 438 may mirror the second surface 440 of the manifold pad 122, in some embodiments.

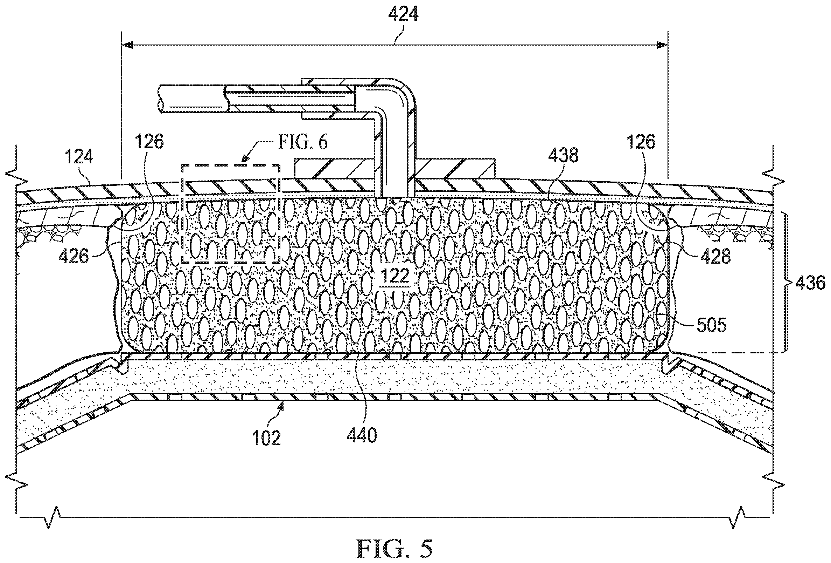

Referring to FIGURES 5-6, some embodiments of the manifold pad 122 may comprise or consist essentially of foam, such as open cell foam. Some manifold pad 122 embodiments may comprise the first surface 438 and the second surface 440, with the thickness 436 of foam extending between the first surface 438 and the second surface 440. In some embodiments, the first surface 438 may be configured to face outward, away from the tissue site (e.g. towards and/or contacting portions of the sealing member 124 over the manifold pad 122), and the second surface 440 may be configured to face towards the tissue site (e.g. toward and/or contacting portions of the treatment device 102 underlying the manifold pad 122). In some embodiments, the foam of the manifold pad 122 may comprise a plurality of elongate, ovoidal or ellipsoidal pores 505 (e.g. cells formed by the cell-structure of the foam). In some embodiments, the foam may comprise open-cell foam. For example, the open-cell foam of the manifold pad 122 may have a cell-structure of open cells or pores, and the ovoidal pores 505 may be formed by the cell-structure. In some embodiments, (not shown) the manifold pad 122 may also include holes, which may provide additional porosity in addition to the porosity provided by the ovoidal pores/cells 505 of the foam of the manifold pad 122. In some embodiments, the manifold pad 122 may be configured for use in an abdominal wound, and may be configured to contract under application of negative pressure in such a way as to laterally close the abdominal wound, typically without significantly drawing down the thickness 436 of the manifold pad 122.

In some embodiments, the manifold pad 122, its foam, and/or the ovoidal pores 505 may be configured to contract (e.g. collapse) in a plane substantially parallel (e.g. approximately +/-10 degrees) to the first surface 438 and/or the second surface 440 upon application of negative pressure. For example, the manifold pad 122 may be configured to contract radially or laterally upon application of negative pressure. In some embodiments, the foam and/or the manifold pad 122 may be configured to preferentially contract in a plane substantially parallel to the first surface 438 and/or the second surface 440 (e.g. radially or laterally) upon application of negative pressure to the manifold pad 122. In some embodiments, the foam of the manifold pad 122 may be configured to preferentially contract in a plane approximately perpendicular to the thickness 436 upon application of negative pressure to the manifold pad 122, for example contracting more in the plane approximately perpendicular to the thickness 436 than in the thickness 436. In some embodiments, the manifold pad 122 may be configured to resist contraction in thickness 436, for example contracting less in thickness 436 than laterally or radially under negative pressure. In some embodiments, the foam of the manifold pad 122 may be configured to be more resistant to contraction of thickness 436 than contraction in a plane substantially parallel to the first surface 438 and/or the second surface 440 (e.g. radially or laterally). In some embodiments, the foam of the manifold 122 may be configured to be more resistant to contraction of the thickness 436 than contraction in a plane approximately perpendicular to the thickness 436. In some embodiments, the manifold pad 122 may be configured to be substantially free of contraction in thickness 436 in a direction extending from the first surface 438 to the second surface 440 under negative pressure. In some embodiments, the manifold pad 122 may be configured so that there may be no significant contraction of the thickness 436 of the manifold pad 122 under applied negative pressure.

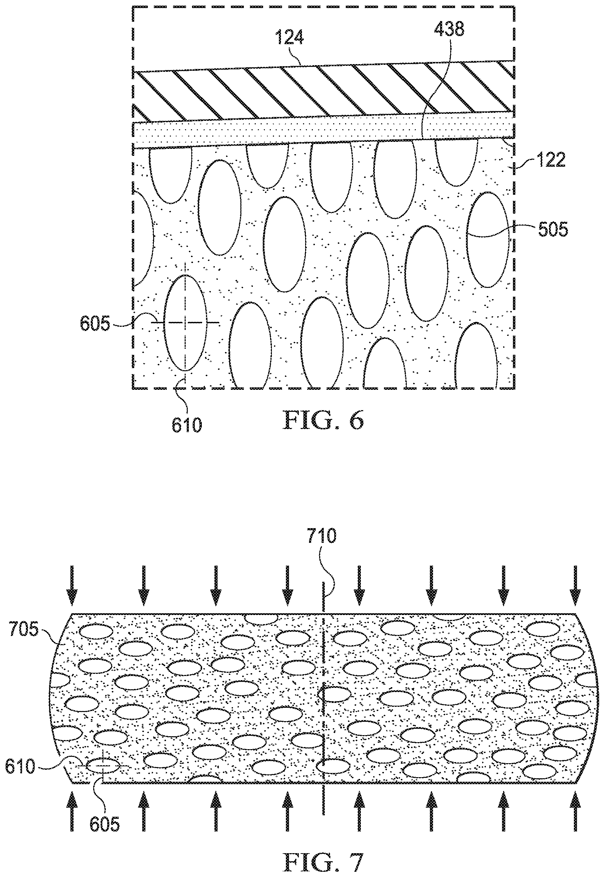

As shown in FIGURE 6, the ovoidal pores 505 may each comprise a major axis 610 and a minor axis 605, with a length of the ovoidal pore 505 along the major axis 610 being longer than a length of the ovoidal pore 505 along the minor axis 605. For example, the major axis 610 of each ovoidal pore 505 may extend longitudinally, and may span the greatest length of the ovoidal pore 505; the corresponding minor axis 605 may extend laterally, and may span the shortest width of the ovoidal pore 505. In some embodiments, for each ovoidal pore 505, the major axis 610 may be oriented substantially perpendicular to the minor axis 605. In some embodiments, the ovoidal pores 505 may be oriented in order to encourage preferential lateral or radial contraction of the manifold pad 122. For example, the minor axis 605 may be oriented to be no more than 44 degrees from being parallel to the first surface 438 and/or the second surface 440 (e.g. oriented in an arc ranging from 44 degrees above parallel to the first and/or second surface to 44 degrees below parallel to the first and/or second surface), and/or the major axis 610 may be oriented to be no more than 44 degrees from being parallel to the thickness 436. Generally, the closer that the minor axis 605 of the ovoidal pores 505 is oriented to parallel with the first surface 438 and/or the second surface 440 (and/or perpendicular to the thickness 436), the more preferential contraction laterally or radially is compared to contraction of thickness 436, upon application of negative pressure to the manifold pad 122. Similarly, the closer that the major axis 610 is oriented to perpendicular with the first surface 438 and/or the second surface 440 (and/or parallel to the thickness 436), the more preferential contraction laterally or radially is compared to contraction of thickness 436, upon application of negative pressure to the manifold pad 122. In some embodiments, the minor axis 605 may be oriented to be no more than 25 degrees, no more than 20 degrees, no more than 15 degrees, or no more than 10 degrees from being parallel to the first surface 438 and/or the second surface 440. Preferably, the major axis 610 may be oriented substantially perpendicular to the first surface 438 and/or the second surface 440, the major axis may be oriented substantially parallel to the thickness, the minor axis may be oriented substantially perpendicular to the thickness, and/or the minor axis 605 may be oriented substantially parallel to the first surface 438 and/or the second surface 440.

In some embodiments, the ovoidal pores 505 may be configured to contract along (e.g. in the direction of) the minor axis 605 upon application of negative pressure to the manifold pad 122. For example, each of the ovoidal pores 505 may be configured to contract from an initial relaxed position to a contracted position in response to application of negative pressure. In some embodiments, the ovoidal pores 505 may be configured to preferentially contract in a plane substantially parallel to the first surface 438 and/or the second surface 440 (e.g. radially or laterally) upon application of negative pressure to the manifold pad 122. In some embodiments, the ovoidal pores 505 may be configured to preferentially contract in a plane approximately perpendicular to the thickness 436, upon application of negative pressure. For example, the ovoidal pores 505 may be configured so that, upon application of negative pressure, each of the ovoidal pores 505 contract more in the direction of the minor axis 605 than in the direction of the major axis 610. In some embodiments, the ovoidal pores 505 may be configured to resist contraction along (e.g. in the direction of) the major axis 610 upon application of negative pressure to the manifold pad 122. In some embodiments, the ovoidal pores 505 may be configured to be more resistant to contraction of along (e.g. in the direction of) the major axis 610 than contraction along (e.g. in the direction of) the minor axis 605. In some embodiments, the ovoidal pores 505 may be configured to undergo no significant contraction along (e.g. in the direction of) the major axis 610 upon application of negative pressure to the manifold pad 122. In some embodiments, the ovoidal pores 505 may be configured to be substantially free of contraction along (e.g. in the direction of) the major axis 610 upon application of negative pressure to the manifold pad 122.

In some embodiments, the foam of the manifold pad 122 may comprise felted foam, which may be formed by a felting process. As described herein, the terms "felting" or "felted" may refer to a porous material, such as the foam of the manifold pad 122, which has been treated or modified to impart a desired property to the porous material. For example, the porous material may be a foam treated or modified to have a desired porosity or density. Any porous foam suitable for felting may be used, including the example foams mentioned herein, such as found in GRANUFOAM^{™} dressing. In general, felting comprises a thermoforming process that permanently compresses a foam to increase the density of the foam while maintaining interconnected pathways. For example, in order to create felted foam, such as felted polyurethane, a foam blank 705 of uncompressed material can be heated to an optimum forming temperature and then compressed.

Felting may be performed by any known methods, which may include applying heat and pressure to a porous material or foam material. Such methods may include compressing the porous material between one or more heated platens or dies (not shown) for a specified period of time and at a specified temperature. As shown in FIGURE 7, the direction (e.g. axis) of compression 710 may be along or parallel to the initial thickness of the foam blank 705 of porous material. The felting process may alter certain properties of the original material, including pore shape and/or size, elasticity, density, and density distribution. For example, struts that define pores in the foam may be deformed during the felting process, resulting in flattened pore shapes. In some embodiments, the felting process may form the ovoidal or ellipsoidal pores or cells. The deformed struts can also decrease the elasticity of the foam. The density of the foam is generally increased by felting. In some embodiments, contact with hot-press platens in the felting process can also result in a density gradient in which the density is greater at the surface and the pores size is smaller at the surface.

A felted foam may be characterized by a firmness factor, which is indicative of the compression of the foam. The firmness factor of a felted foam can be specified as the ratio of original thickness to final thickness. A compressed or felted foam may have a firmness factor greater than 1. The degree of compression may affect the physical properties of the felted foam. For example, felted foam typically may have an increased effective density compared to a foam of the same material that is not felted. The felting process can also affect fluid-to-foam interactions. For example, as the density increases, compressibility or collapse may decrease in some directions. There is a general linear relationship between firmness level, density, pore size (or pores per inch) and compressibility. For example, foam found in a GRANUFOAM^{™} dressing that is felted to a firmness factor of 3 will show a three-fold density increase and compress to about a third of its original thickness.

The period of time of compression may range from 10 minutes up to 24 hours, though the time period may be more or less depending on the specific type of porous material used. Further, in some examples, the temperature may range between 120°C to 260°C. Generally, the lower the temperature of the platen, the longer a porous material must be held in compression. After the specified time period has elapsed, the pressure and heat will form a felted structure or surface on or through the porous material or a portion of the porous material. The felted structure may be comparatively smoother than any unfinished or non-felted surface or portion of the porous material. Further, the pores in the felted structure may be smaller than the pores throughout any unfinished or non-felted surface or portion of the porous material. In some examples, the felted structure may be applied to all surfaces or portions of the porous material. Further, in some examples, the felted structure may extend into or through an entire thickness of the porous material such that the all of the porous material is felted. Typically, the entire manifold pad 122 may be felted to the same firmness factor throughout, although in other embodiments the firmness factor may vary throughout the manifold pad 122.

In some embodiments, the felted foam for the manifold pad 122 may be formed from a foam blank 705 of open cell foam, such as polyurethane foam, having an initial (e.g. pre-felted) density of approximately 1.3-1.6 lb/ft³, a free volume of approximately 90% or more, an average number of pores per inch of approximately 40-50, an average pore size of approximately 400-600 micron, a 25% compression load deflection of at least 0.35 pounds per square inch, and/or a 65% compression load deflection of at least 0.43 pounds or square inch. The felted foam may have an axis (e.g. direction) of compression 710. As shown in FIGURE 7, the foam blank 705 may be compressed and/or felted in the direction of the axis of compression 710 (e.g. parallel to the axis of compression 710), to form the foam with ovoidal pores 505. In some embodiments, the minor axis 605 of the ovoidal pores 505 may be substantially parallel to the axis of compression 710 and/or the major axis 610 of the ovoidal pores 505 may be substantially perpendicular to the axis of compression 710. In some embodiments, the felted foam may be compressed (e.g. felted) to have a firmness factor of 2-7, 3-7, 2-5, 3-5, 2-3, or 5-7.

In some embodiments, the manifold pad 122 may comprise or consist essentially of an open-cell foam having a free volume in a range of about 13% to about 45%, a density of about 2.6-112 lb/ft³, about 80-350 pores per inch on average (e.g., as measured in the direction/axis of compression 710), and/or average pore size of about 57-300 micron (e.g., as measured in the direction/axis of compression 710); in some embodiments, a foam blank 705 (for example, similar to that described above) may be felted to obtain foam with these characteristics. In some embodiments, the manifold pad 122 may comprise or consist essentially of an open-cell foam having a free volume in a range of about 18% to about 45%, a density of about 2.6-8.0 lb/ft3 (1lb/ft³ = 16.0185 kg/m³), about 80-250 pores per inch on average (e.g., as measured in the direction/axis of compression 710, 1 inch = 2.54 cm), and/or average pore size of about 80-300 micron (e.g., as measured in the direction/axis of compression 710); in some embodiments, a foam blank 705 (for example, similar to that described above) may be felted to obtain foam with these characteristics. In some embodiments, the manifold pad 122 may comprise or consist essentially of an open-cell foam having a free volume in a range of about 18% to about 30%, a density of about 3.9-8.0 lb/ft³, about 120-250 pores per inch on average (e.g., as measured in the direction/axis of compression 710), and/or average pore size of about 80-200 micron (e.g., as measured in the direction/axis of compression 710); in some embodiments, a foam blank 705 (for example, similar to that described above) may be felted to obtain foam with these characteristics. In some embodiments, the manifold pad 122 may comprise or consist essentially of an open-cell foam having a free volume in a range of about 30% to about 45%, a density of about 2.6-4.8 lb/ft³, about 80-150 pores per inch on average (e.g., as measured in the direction/axis of compression 710), and/or average pore size of about 133-300 micron (e.g., as measured in the direction/axis of compression 710); in some embodiments, a foam blank 705 (for example, similar to that described above) may be felted to obtain foam with these characteristics. In some embodiments, the manifold pad 122 may comprise or consist essentially of an open-cell foam having a free volume in a range of about 30-33%, a density of about 3.9-4.8 lb/ft³, about 120-150 pores per inch on average (e.g., as measured in the direction/axis of compression 710), and/or average pore size of about 133-200 micron (e.g., as measured in the direction/axis of compression 710); in some embodiments, a foam blank 705 (for example, similar to that described above) may be felted to obtain foam with these characteristics.

In some embodiments, the felted foam may be configured and/or oriented with the ovoidal pores 505 having their major axis 610 approximately parallel to the thickness 436, which may result in improved contraction characteristics compared to unfelted foam of the same type and thickness. For example, the felted foam may be more resistant to contraction of thickness, under applied negative pressure, than similar unfelted foam and/or may provide greater lateral contraction, under applied negative pressure, than similar unfelted foam. See for example the following experimental data.

Samples were provided: three unfelted GRANUFOAM^{™} blocks with starting size of 25mm (height) by 25 mm (width) and approximately spherical pores, three 3X felted (e.g. firmness factor of 3) GRANUFOAM^{™} blocks with starting size of 25mm (height) by 25mm (width) and ovoidal pores oriented with the major axis approximately parallel to the thickness (e.g. height), and three 5X felted (e.g. firmness factor of 5) GRANUFOAM^{™} blocks with starting size of 25 mm (height) by 25 mm (width) and ovoidal pores oriented with the major axis approximately parallel to the thickness (e.g. height). Negative pressure (e.g. approximately -125 mmHg) was applied to all 22 three samples, and the height and width measurements under applied negative pressure were recorded as follows:

| | Sample 1 | | Sample 2 | | Sample 3 | | Average | |
|---|---|---|---|---|---|---|---|---|
| | (h) | (w) | (h) | (w) | (h) | (w) | (h) | (w) |
| Unfelted GRANUFOAM^{™} | 4.53 mm | 24.56 mm | 3.98 mm | 24.83 mm | 4.47 mm | 24.33 mm | 4.33 mm | 24.57 mm |
| 3X Felted with major axis parallel to thickness | 14.25 mm | 15.84 mm | 14.11 mm | 14.03 mm | 15.62 mm | 13.80 mm | 14.66 mm | 14.56 mm |
| 5X Felted with major axis parallel to thickness | 20.08 mm | 18.03 mm | 21.13 mm | 17.81 mm | 20.56 mm | 17.69 mm | 20.59 mm | 17.84 mm |

In some embodiments, the felted foam with ovoidal pores oriented with the major axis approximately parallel to the thickness (e.g. height) may draw down in thickness under applied negative pressure approximately ½ or less the amount compared to similar unfelted foam. For example, the felted foam with ovoidal pores oriented with the major axis approximately parallel to the thickness may draw down in thickness under applied negative pressure approximately ½ - 1/5 the amount compared to similar unfelted foam. In some embodiments, the felted foam with ovoidal pores oriented with the major axis approximately parallel to the thickness may have lateral contraction under applied negative pressure of approximately 16 or more times greater than the amount compared to similar unfelted foam. For example, the felted foam with ovoidal pores oriented with the major axis approximately parallel to the thickness may have lateral contraction under applied negative pressure which is approximately 16 - 24 times greater than the amount compared to similar unfelted foam.

In some embodiments, resistance to contraction in thickness due to orientation of the ovoidal pores may maintain a surface area that will transmit a higher lateral closure force under applied negative pressure as compared to the closure force from similar unfelted foam. For example, 3X felted foam (e.g. foam with a firmness factor of 3) with ovoidal pores oriented approximately parallel to the thickness may have a lateral closure force which is approximately 30-70% better (e.g. greater) than similar unfelted foam, under applied negative pressure. Similarly, if a specific lateral closure force is desired under applied negative pressure, then that amount of lateral closure force may be generated using less negative pressure when felted foam with ovoidal pores oriented approximately parallel to the thickness is used instead of similar unfelted foam.

Some dressing embodiments, as shown in FIGURES 5-6 for example, may comprise a foam manifold pad 122 similar to those described herein. In some embodiments, the manifold pad 122 may be configured for use with an abdominal wound, and may be configured to contract under application of negative pressure in such a way as to laterally close the abdominal wound without significantly drawing down the thickness 436 of the manifold pad 122. In some embodiments, the dressing may further comprise a treatment device 102, which may be configured as a viscera protection layer to provide separation between the abdominal wall and the viscera and protect abdominal contents. In some embodiments, the treatment device 102 may have a film layer and may be configured to channel fluid radially under applied negative pressure. In some embodiments, the treatment device 102 may comprise a central hub and a plurality of elongate members extending outward from the central hub. In some embodiments, the plurality of elongate members may comprise 4-8 elongate members, which may be evenly spaced around the central fluid hub. In some embodiments, each elongate member may be in fluid communication with the central fluid hub. In some embodiments, the film layer may comprise a plurality of channels configured to allow radial fluid flow (e.g. flow from the elongate members to the central fluid hub). In some embodiments, the manifold pad 122 may be configured to be positioned proximate to and/or contacting the central fluid hub (e.g. between the central fluid hub of the treatment device 102 and the sealing member 124).

In some embodiments, the treatment device 102 may further comprise a treatment manifold, which may be attached to the film layer. In some embodiments, the treatment manifold may comprise open-cell foam. In some embodiments, the treatment manifold may be enveloped or encapsulated by the film layer, which may be a non-adherent drape with a plurality of fenestrations. In some embodiments, the treatment manifold may comprise a central connection manifold member and a plurality of leg manifold members extending outward from the central connection manifold member. In some embodiments, there may be 4-8 leg manifold members, which may be evenly spaced around the central connection manifold member. In some embodiments, each of the leg manifold members may be in fluid communication with the central connection manifold member. Some dressing embodiments may further comprise a sealing member 124 configured to cover the manifold pad 122 and the treatment device 102 and to provide a pneumatic seal relative to the tissue site. In some embodiments, the sealing member 124 may be configured to be disposed over the treatment device 102 and manifold pad 122 to create a sealed space on the tissue site. In some embodiments, the manifold pad 122 may be configured so that the first surface 438 faces towards and/or contacts the portions of the sealing member 124 disposed over the manifold pad 122, and the second surface 440 faces towards and/or contacts the portions of the treatment device 102 underlying the manifold pad 122. In some embodiments, the manifold pad 122 may be configured to distribute applied negative pressure to the treatment device 102 (and thereby to the tissue site). In some embodiments, the treatment device 102 may comprise a single film layer, and the single film layer may be attached to a surface of the treatment manifold. In some embodiments, the film layer may comprise a plurality of fenestrations. In some embodiments, the treatment manifold may comprise closed-cell foam, which may have a plurality of channels and/or through-passages configured for distribution of applied negative pressure and/or drawing fluid (such as exudate) radially inward. In some embodiments, the treatment device 102 may be similar to those described in more detail with respect to FIGURES 1A-4.

In some embodiments, each of the ovoidal pores 505 may be configured to be oriented with the minor axis 605 substantially parallel to the portion of the sealing member 124 over the manifold pad 122. In some embodiments, each of the ovoidal pores 505 may be configured to be oriented with the minor axis 605 substantially parallel to the portion of the treatment device 102 underlying the manifold pad 122. In some embodiments, each of the ovoidal pores 505 may be configured to be oriented with the minor axis 605 substantially parallel to a portion of the tissue site underlying the manifold pad 122.

System embodiments may comprise a dressing similar to those described herein, and a negative-pressure source fluidly coupled to the dressing. In some embodiments, the negative-pressure source may fluidly couple to the sealed space formed by the dressing. For example, the negative-pressure source may fluidly communicate with the manifold pad 122 through the sealing member 124, and the applied negative pressure may be distributed through the manifold pad 122, through the treatment device 102, to the tissue site. In some embodiments, the dressing may be used on a tissue site with an incision, and the application of negative pressure to the manifold pad 122 may draw the incision or wound edges closer together to promote closure of the wound tissue site.

Method embodiments, for forming a manifold pad 122 or dressing, may comprise: providing a blank of foam having a plurality of pores; altering the foam blank 705 to form foam, wherein altered pores of the foam each are ovoidal with a major axis 610 and a minor axis 605; and shaping the foam to form a manifold pad 122 having a first surface 438, a second surface 440, and a thickness 436 of foam extending between the first surface 438 and the second surface 440. In some embodiments, the minor axis 605 may be substantially parallel to the first surface 438 and/or approximately perpendicular to the thickness. In some embodiments, altering the foam blank 705 may comprise compressing the foam blank 705 along an axis (e.g. direction) of compression 710 to permanently deform the plurality of pores to form the foam with ovoidal pores 505 having the major axis 610 substantially perpendicular to the axis of compression 710 and the minor axis 605 substantially parallel to the axis of compression 710. FIGURE 7 illustrates such alteration of the foam blank 705. In some embodiments, the foam may be heated as it is compressed.

In some embodiments, shaping the foam may comprise cutting the foam approximately perpendicular to the axis of compression 710. In some embodiments, shaping the foam may comprise cutting the foam to form the first surface 438 substantially parallel to the minor axis 605 and the major axis 610 substantially perpendicular to the first surface 438. In some embodiments, shaping the foam may comprise rotating the foam at least 46 degrees, at least 65 degrees, at least 70 degrees, or at least 80 degrees before cutting the foam through the thickness (e.g. substantially perpendicular to the axis of compression). Generally, the closer to 90 degrees that the foam is rotated, the more preferential contraction laterally or radially is compared to contraction of thickness; so in some embodiments, shaping the foam may comprise rotating the foam approximately 90 degrees (e.g. rotating the axis of compression 710 approximately 90 degrees after compression), and then cutting the foam through the thickness 436 (e.g. substantially perpendicular to the rotated axis of compression). In some embodiments, the axis of compression 710 may be rotated approximately 90 degrees prior to cutting, with cutting then occurring through the thickness and/or approximately perpendicular to the rotated axis of compression 710. In some embodiments, altering the foam blank 705 may comprise felting the foam blank 705 to a firmness factor of 2-7, 2-5, 3-5, or approximately 3 along the axis of compression 710 so that the foam is formed with ovoidal pores 505 having the major axis 610 substantially perpendicular to the axis of compression 710 and the minor axis 605 substantially parallel to the axis of compression 710. In some embodiments, the foam blank 705 may have an original thickness, and felting the foam blank 705 may comprise heating and compressing the foam blank 705 to approximately ½ - 1/7, ½-1/5, or 1/3-1/5 of the original thickness. In some embodiments, multiple foam blanks may be provided and altered (e.g. by felting) to form ovoidal pores, and the plurality of altered foam blanks (e.g. the plurality of foams resulting from the alteration process) may then be joined, for example by lamination. Joining the plurality of foams together may include orienting the plurality of foams so that the ovoidal pores in all of the plurality of foams are oriented approximately the same direction (e.g. with major axes parallel). In some embodiments, the pre-felted or pre-compressed foam blank 705 may have a density of approximately 1.3-1.6 lb/ft³, a free volume of approximately 90% or more, an average number of pores per inch of approximately 40-50, an average pore size of approximately 400-600 micron, a 25% compression load deflection of at least 0.35 pounds per square inch (1 psi = 6.89476 kPa), and/or a 65% compression load deflection of at least 0.43 pounds or square inch.

In some embodiments, the method may further comprise providing a treatment device 102 having a film layer and configured to channel fluid radially under applied negative pressure. In some embodiments, providing a treatment device 102 may comprise forming a treatment manifold having a central connection manifold member and a plurality of leg manifold members extending outward from the central connection manifold member, with each of the leg manifold members being in fluid communication with the central connection manifold member. In some embodiments, providing a treatment device 102 may further comprise attaching the film layer to the treatment manifold. In some embodiments, the film layer may encapsulate the treatment manifold.

For treating a tissue site, for example using a manifold pad 122, treatment device 102, dressing and/or system similar to those described herein, exemplary methods may comprise: applying a treatment device 102, similar to those described herein, to the tissue site; applying a manifold pad 122, similar to those described herein, over the treatment device 102; and applying a sealing member 124 over the manifold pad 122 to form a sealed space on the tissue site. In some embodiments, the manifold pad 122 may be applied with the first surface 438 substantially parallel to a portion of the tissue site underlying the manifold pad 122, with the ovoidal pores 505 oriented with the major axis 610 substantially perpendicular to the first surface 438, with the minor axis 605 substantially parallel to the first surface 438, with the minor axis 605 substantially parallel to the portion of the tissue site underlying the manifold pad with the minor axis 605 substantially parallel to the portions of the treatment device 102 underlying the manifold pad and/or with the minor axis 605 substantially parallel to a portion of the sealing member 124 disposed over the manifold pad 122. Some embodiments may further comprise applying negative pressure to the manifold pad 122, and responsive to application of the negative pressure, contracting the manifold pad 122 in a plane substantially parallel to the first surface 438 without significantly drawing down (e.g. reduction) of the thickness 436. In some embodiments, applying negative pressure may comprise fluidly coupling a negative-pressure source to the manifold pad 122 through the sealing member 124.

Another illustrative embodiment for using an open-cavity, reduced-pressure treatment device or system according to this disclosure will now be presented. The system is particularly suitable for temporary bridging of abdominal wall openings where primary closure may not be readily possible and or repeat abdominal entries are necessary. The illustrative system described here may be used with open abdominal wounds, with exposed viscera, including but not limited to abdominal compartment syndrome. Before applying the system, typically hemostasis should be achieved.

In deploying the open-cavity, reduced-pressure treatment system, the reduced-pressure treatment device preferably covers all exposed viscera and preferably separates completely the viscera from contact with the abdominal wall. For example, the lower surface of the reduced pressure treatment device, such as drape 148, can be sized and shaped to permit coverage. The reduced-pressure treatment device may be placed over the omentum or exposed internal organs, and carefully tucked between the abdominal wall and internal organs. In doing so, the healthcare provider may use the reduced-pressure treatment device to completely separate the abdominal wall from the internal organs.

To prepare for deployment of the system, any sharp edges or bone fragments are eliminated from wound area or covered. The abdominal wound is irrigated and the periwound area cleaned. The periwound tissue at the epidermis is typically dried before further application.

The reduced-pressure treatment device is then sized by determining the appropriate size and cutting. The reduced-pressure treatment device is initially unfolded in a sterile field. Either side of the reduced-pressure treatment device may be placed on the omentum or viscera. The reduced-pressure treatment device is gently placed over the open abdominal cavity. The orientation of the reduced-pressure treatment device for the specific application is determined. If the reduced-pressure treatment device will be placed around tubes, drains or the falciform ligament, the reduced-pressure device is cut only between the plurality of encapsulated leg members. The reduced-pressure treatment device is placed in the proper orientation before cutting.

The reduced-pressure treatment device is then folded to size and used that way or may be cut. The healthcare provider holds the reduced-pressure treatment device by the edge and slightly lifts the reduced-pressure treatment device. The reduced-pressure treatment device is slowly lowered into the paracolic gutter with one hand and the other hand is used to gently and evenly work the reduced-pressure treatment device down. The healthcare provider folds any excess portions of the reduced-pressure treatment device up and over onto itself. The healthcare provider continues to place the reduced-pressure treatment device between abdominal wall and internal organs throughout the abdominal compartment. The healthcare provider preferably provides full coverage of all viscera. The reduced-pressure treatment device may then be cut as needed for sizing outside of the wound.

To size the device, the reduced-pressure treatment device can be cut through center of one of the large manifold squares, or leg modules, using sterile scissors. In this illustrative embodiment, the cut is not made through the manipulation zone, but through the leg module. The healthcare provider then pinches the remaining half of the foam square, or leg module, and the adjacent, inboard manipulation zone through the encapsulating member with one hand and pulls the manifold material. The manifold material in the leg module and the manipulation zone will separate at the next square, or leg module. This will ensure that edges of the reduced-pressure treatment device cover the otherwise exposed manifold edge. The manifold material, *e.g.*, foam, preferably does not contact organs.

The manifold pad that is to be placed on top of the central connection member is next prepared. In this embodiment, the manifold pad may be a perforated foam manifold that has perforations to help tear the manifold to the desired size. The manifold pad preferably fits directly over the reduced-pressure treatment device while still being in contact with wound edges. The manifold pad generally should not contact intact skin. Two or more manifolds may be used in some instances. Then, the sized manifold pad is gently placed into the wound cavity over the reduced-pressure treatment device. The healthcare provider preferably takes care to avoid the manifold pad going below the level of the abdominal incision or wound.

A drape, or over-drape or sealing member, is then applied. To apply the drape, a backing is removed from an adhesive layer on one side of the drape and the drape is applied. The drape covers the manifold and a portion of intact epidermis. Preferably the drape covers at least an 8-10 centimeter border of intact periwound tissue. Additional drape material may be added to seal any difficult areas.

The reduced-pressure interface, or interface pad, is then added. The healthcare provider chooses an application site. The site is chosen to optimize fluid flow as well as to facilitate easy tubing positioning. The healthcare provider pinches the drape and cuts a 2.5 cm hole (preferably not a slit) through the drape. The interface pad, which has a central disc and a surrounding outer adhesive skirt, is applied. The interface pad is applied by removing backing layers on an inward-facing surface of the interface pad to expose an adhesive. The interface pad opening in the central disc is placed directly over the hole in the drape. Pressure is gently applied on the central disc and the outer skirt to ensure complete adhesion of the interface pad. One or more stabilization layers may then be removed from a first side of the skirt. The system is now ready for the application of reduced pressure.

The systems, apparatuses, and methods described herein may provide significant advantages. For example, disclosed manifold pads or dressings may distribute negative pressure to a tissue site, which may facilitate removal of fluids, such as exudate, from the tissue site. Some embodiments of the manifold pad or dressing may be configured to draw edges of an incision at the tissue site towards one another. For example, application of negative pressure to the manifold pad may provide lateral closing force to the incision. Some embodiments of the pad may also be configured to resist collapse of thickness upon application of negative pressure. These and other advantages may arise from the disclosure.

If something is described as "exemplary" or an "example", it should be understood that refers to a non-exclusive example. The terms "about" or "approximately" or the like, when used with a number, may mean that specific number, or alternatively, a range in proximity to the specific number as understood by persons of skill in the art field (for example, +/-10%). Use of broader terms such as "comprises", "includes", and "having" should be understood to provide support for narrower terms such as "consisting of", "consisting essentially of", and "comprised substantially of'. Use of the term "optionally", "may", "might", "possibly", "could", "can", "would", "should", "preferably", "typically", "often" and the like with respect to any element, component, feature, characteristic, etc. of an embodiment means that the element, component, feature, characteristic, etc. is not required, or alternatively, the element, component, feature, characteristic, etc. is required, both alternatives being within the scope of the embodiment(s). Such element, component, feature, characteristic, etc. may be optionally included in some embodiments, or it may be excluded (e.g. forming alternative embodiments, all of which are included within the scope of disclosure). Section headings used herein are provided for consistency and convenience, and shall not limit or characterize any invention(s) set out in any claims that may issue from this disclosure. If a reference numeral is used to reference a specific example of a more general term, then that reference numeral may also be used to refer to the general term (or vice versa).

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the treatment device, manifold pad, and/or sealing member may be eliminated or separated from other components for manufacture or sale. In other example configurations, the controller may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described in the context of some embodiments may also be omitted, combined, or replaced by alternative features serving the same, equivalent, or similar purpose without departing from the scope of the invention defined by the appended claims. Also, features, elements, and aspects described with respect to a particular embodiment may be combined with features, elements, and aspects described with respect to one or more other embodiments.

## Claims

1. A foam manifold pad, comprising:
a first surface (438);
a second surface (440); and
a thickness (436) of foam extending between the first surface (438) and the second surface (440);
wherein:
the foam of the manifold pad (122) comprises a plurality of joined felted open-cell foam blanks, each foam blank comprising a plurality of ovoidal pores; and
the manifold pad (122) is configured to contract in a plane substantially parallel to the first surface (438) upon application of negative pressure;
wherein the ovoidal pores are configured to contract in a direction substantially parallel to the first surface (438) upon application of negative pressure to the manifold pad (122).

2. The manifold pad of claim 1, wherein the manifold pad (122) is configured to be substantially free of contraction in thickness in a direction extending from the first surface (438) to the second surface (440) under negative pressure.

3. The manifold pad of claim 1, wherein the manifold pad (122) is configured to contract more radially or laterally than in thickness (438) under applied negative pressure.

4. The manifold pad of claim 1, wherein:
the ovoidal pores each comprise a major axis and a minor axis;
the major axis is oriented substantially perpendicular to the first surface (438);
the minor axis is oriented substantially parallel to the first surface (438);
for each ovoidal pore, the major axis is substantially perpendicular to the minor axis; and
for each ovoidal pore, a length along the major axis is longer than a length along the minor axis.

5. The manifold pad of claim 4, wherein the ovoidal pores are configured so that, upon application of negative pressure, the ovoidal pores contract more in the direction of the minor axis than in the direction of the major axis.

6. The manifold pad of claim 1, wherein the felted foam comprises a firmness factor of 2-7.

7. The manifold pad of claim 6, wherein the ratio of contraction of the minor axis of the ovoidal pores to contraction of the major axis, under applied negative pressure, is greater than 1.

## Patentansprüche

1. Ein Schaumstoffverteilerpad, aufweisend:
eine erste Oberfläche (438);
eine zweite Oberfläche (440); und
eine Dicke (436) von Schaumstoff, der sich zwischen der ersten Oberfläche (438) und der zweiten Oberfläche (440) erstreckt;
wobei:
der Schaumstoff des Verteilerpads (122) eine Mehrzahl von verbundenen filzigen offenzelligen Schaumstoffrohlingen aufweist, jeder Schaumstoffrohling aufweisend eine Mehrzahl von ovoidalen Poren; und
das Verteilerpad (122) konfiguriert ist, um bei Anwendung eines Unterdrucks in einer Ebene im Wesentlichen parallel zu der ersten Oberfläche (438) zu kontrahieren;
wobei die ovoidalen Poren konfiguriert sind, um bei Anwendung eines Unterdrucks an das Verteilerpad (122) in einer Richtung im Wesentlichen parallel zu der ersten Oberfläche (438) zu kontrahieren.

2. Das Verteilerpad nach Anspruch 1, wobei das Verteilerpad (122) konfiguriert ist, um im Wesentlichen frei von Kontraktion in Dicke in einer Richtung zu sein, die sich von der ersten Oberfläche (438) zu der zweiten Oberfläche (440) unter Unterdruck erstreckt.

3. Das Verteilerpad nach Anspruch 1, wobei das Verteilerpad (122)
konfiguriert ist, um mehr radial oder lateral als in Dicke (438) unter angewendeten Unterdruck zu kontrahieren.

4. Das Verteilerpad nach Anspruch 1, wobei:
die ovoidalen Poren jeweils eine Hauptachse und eine Nebenachse aufweisen;
die Hauptachse im Wesentlichen senkrecht zu der ersten Oberfläche (438) ausgerichtet ist;
die Nebenachse im Wesentlichen parallel zu der ersten Oberfläche (438) ausgerichtet ist;
für jede ovoidale Pore die Hauptachse im Wesentlichen senkrecht zu der Nebenachse ist; und
für jede ovoidale Pore eine Länge entlang der Hauptachse länger als eine Länge entlang der Nebenachse ist.

5. Das Verteilerpad nach Anspruch 4, wobei die ovoidalen Poren konfiguriert sind, sodass die ovoidalen Poren bei Anwendung von Unterdruck mehr in der Richtung der Nebenachse kontrahieren als in der Richtung der Hauptachse.

6. Das Verteilerpad nach Anspruch 1, wobei der filzige Schaumstoff einen Festigkeitsfaktor von 2-7 aufweist.

7. Das Verteilerpad nach Anspruch 6, wobei das Verhältnis von Kontraktion der Nebenachse der ovoiden Poren zu Kontraktion der Hauptachse unter angewendeten Unterdruck größer als 1 ist.

## Revendications

1. Tampon collecteur en mousse, comprenant :
une première surface (438) ;
une seconde surface (440) ; et
une épaisseur (436) de mousse s'étendant entre la première surface (438) et la seconde surface (440) ;
dans lequel :
la mousse du tampon collecteur (122) comprend une pluralité d'ébauches en mousse à cellules ouvertes feutrées réunies, chaque ébauche en mousse comprenant une pluralité de pores ovoïdaux ; et
le tampon collecteur (122) est conçu pour se contracter dans un plan sensiblement parallèle à la première surface (438) lors d'une application de pression négative ;
dans lequel les pores ovoïdaux sont conçus pour se contracter dans une direction sensiblement parallèle à la première surface (438) lors de l'application de pression négative sur le tampon collecteur (122).

2. Tampon collecteur selon la revendication 1, dans lequel le tampon collecteur (122) est conçu pour être sensiblement dépourvu de contraction en épaisseur dans une direction s'étendant de la première surface (438) à la seconde surface (440) sous la pression négative.

3. Tampon collecteur selon la revendication 1, dans lequel le tampon collecteur (122) est conçu pour se contracter davantage radialement ou latéralement qu'en épaisseur (438) sous la pression négative appliquée.

4. Tampon collecteur selon la revendication 1, dans lequel :
les pores ovoïdaux comprennent respectivement un axe majeur et un axe mineur ;
l'axe majeur est orienté sensiblement perpendiculairement à la première surface (438) ;
l'axe mineur est orienté sensiblement parallèlement à la première surface (438) ;
pour chaque pore ovoïdal, l'axe majeur est sensiblement perpendiculaire à l'axe mineur ; et
pour chaque pore ovoïdal, une longueur le long de l'axe majeur est plus longue qu'une longueur le long de l'axe mineur.

5. Tampon collecteur selon la revendication 4, dans lequel les pores ovoïdaux sont conçus de telle sorte que, lors de l'application d'une pression négative, les pores ovoïdaux se contractent davantage dans la direction de l'axe mineur que dans la direction de l'axe majeur.

6. Tampon collecteur selon la revendication 1, dans lequel la mousse feutrée comprend un facteur de fermeté allant de 2 à 7.

7. Tampon collecteur selon la revendication 6, dans lequel le rapport entre la contraction de l'axe mineur des pores ovoïdaux et la contraction de l'axe majeur, sous la pression négative appliquée, est supérieur à 1.
